# Europäisches Patentamt

## European Patent Office

## Office européen des brevets

(19)

(11) Veröffentlichungsnummer: **0 092 761**
**B1**

(12) **EUROPÄISCHE PATENTSCHRIFT**

(45) Veröffentlichungstag der Patentschrift:
**13.07.88**

(21) Anmeldenummer: **83103720.5**

(22) Anmeldetag: **18.04.83**

(51) Int. Cl.⁴: **C 12 P 19/04**, C 12 P 39/00,
C 12 N 1/20, B 01 J 19/06 //
C12R1/01, C12R1/38

(54) **Mikrobielle Polysaccharide, Verfahren zu ihrer Herstellung, dafür geeignete Mikroorganismen und Verwendung der Polysaccharide.**

(30) Priorität: **22.04.82 DE 3214953**

(43) Veröffentlichungstag der Anmeldung:
**02.11.83 Patentblatt 83/44**

(45) Bekanntmachung des Hinweises auf die Patenterteilung:
**13.07.88 Patentblatt 88/28**

(84) Benannte Vertragsstaaten:
**AT BE CH DE FR GB IT LI LU NL SE**

(56) Entgegenhaltungen:
**EP - A - 0 040 445**
**GB - A - 293 015**
**GB - A - 1 061 043**
**GB - A - 1 348 304**
**US - A - 3 320 136**
**US - A - 3 406 114**
**US - A - 4 233 438**
**US - A - 4 302 542**

**CHEMICAL ABSTRACTS, Band 83, Nr. 25, 22. Dezember 1975, Seite 179, Nr. 203588j, Columbus, Ohio, US; TAPIL'SKAYA, N.V. et al.: "Polysaccharides of associative cultures of soil amebas and Azotobacter" & BIOL. AKT. VESHCHESTVA MIKROORG. IKH ISPOL'Z, 1974, 120-5**

(73) Patentinhaber: **HOECHST AKTIENGESELLSCHAFT,**
**Postfach 80 03 20, D-6230 Frankfurt am Main 80 (DE)**

(72) Erfinder: **Voelskow, Hartmut, Dr., Eschenstrasse 24,**
**D-6234 Hattersheim am Main (DE)**
Erfinder: **Schlingmann, Merten, Dr., Schneidhainer Strasse 32a, D-6240 Königstein/Taunus (DE)**

## Beschreibung

Mikrobielle Polysaccharide wie Xanthan Gum finden vielseitige Anwendung als Viskositätsregulatoren, wie beispielsweise bei der Erdölförderung, als Zusatz zu Kunststoffdispersionen und für Farbmittelzubereitungen.

Die Erfindung betrifft extrazelluläre Polysaccharide, enthaltend Glucose und Galactose im Molverhältnis von 4 : 1 bis 8 : 1 als Hauptkomponenten und als Nebenkomponenten etwa 4–9 Gew.-% Pyruvat, etwa 5–15 Gew.-% Succinat, etwa 0,5–7 Gew.-% Rhamnose und etwa 0,2–5 Gew.-% Mannose, erhältlich durch Fermentation einer Mischkultur aus zwei oder mehreren Mikroorganismen aus der Gruppe Agrobacterium, Alcaligenes, Arthrobacter, Aureobasidium, Azotobacter, Bacillus, Corynebacterium, Erwinia, Hansenula, Klebsiella, Leuconostoc, Methylococcus, Methylocystis, Methylomonas, Nocardia, Pseudomonas, Sclerotium, Streptomyces und Xanthomonas, von denen mindestens einer auch in Reinkultur ein Polysaccharid produziert.

Ferner betrifft die Erfindung das Verfahren zur Herstellung der genannten Polysaccharide sowie dessen Verwendung als Viskositätsregulator.

Die Erfindung wird im folgenden detailliert, insbesondere in ihren bevorzugten Ausführungsformen, beschrieben.

Die Mischkulturen können – abhängig von den eingesetzten Mikroorganismen – gleich nach dem Mischen in das Produktionsmedium geimpft werden oder werden über einige Generationen zunächst als Mischkultur gehalten, bevor sie in das Produktionsmedium eingebracht werden. Das im Einzelfall zweckmässige Vorgehen ist anhand von Vorversuchen leicht zu ermitteln.

Das optimale Medium für die Mischkultur kann sich von den optimalen Medien für die Reinkultur unterscheiden. Das Auffinden der zweckmässigsten Medien ist auch hier dem Fachmann anhand einfacher Vorversuche ohne erfinderisches Zutun möglich.

Im Vergleich zu den Polysacchariden, die von den jeweils eingesetzten Reinkulturen produziert werden, zeichnen sich die erfindungsgemässen Biopolymere durch verbesserte Eigenschaften aus. So zeigen sie in wässriger und/oder wässriger salzhaltiger Lösung eine Viskosität, die deutlich über derjenigen liegt, die mit gleichen Mengen der Polysaccharide erhalten werden, die mit den entsprechenden Reinkulturen gewonnen wurden. Entsprechende Viskositätsmessungen an den Lösungen der Produkte können auch als Kriterium für die Auswahl der Kulturmedien herangezogen werden.

Für die erfindungsgemäss einzusetzenden Mischkulturen können Arten folgender Gattungen von Bakterien oder Pilzen verwendet werden:

Agrobacterium, Alcaligenes, Arthrobacter, Aureobasidium, Azotobacter, Bacillus, Corynebacterium, Erwinia, Hansenula, Klebsiella, Leuconostoc, Methylococcus, Methylocystis, Methylomonas, Nocardia, Pseudomonas, Sclerotium, Streptomyces und Xanthomonas.

Das (Volumen)-Verhältnis der für die Mischkultur eingesetzten Reinkulturen kann von 1 : 1 bis 1 : 10 000 betragen und liegt vorzugsweise im Bereich von 1 : 1 bis 1 : 100, insbesondere von 1 : 1 bis 1 : 10.

Vorzugsweise enthält die Mischkultur eine Reinkultur der Gattung Pseudomonas, insbesondere den Stamm Pseudomonas maltophilia, der bei der Deutschen Sammlung von Mikroorganismen unter der Nummer DSM 2130 hinterlegt ist. Dieser Stamm sowie seine Mutanten, die ebenfalls in Mischkulturen extrazelluläre Polymere mit vorteilhaften Eigenschaften bilden, sind weiterhin Gegenstand der Erfindung.

Da viele Pseudomonas-Kulturen allein kein brauchbares Polymer und zum Teil nur geringe Mengen niedrigviskoser Schleime bilden, ist es überraschend, dass Mischkulturen, die solche Stämme enthalten, Polymere mit hervorragenden Eigenschaften produzieren. Bei der Verwendung von Pseudomonas-Kulturen werden häufig Mischkulturen erhalten, die über zahlreiche Generationen stabil sind. Die Ausscheidung der erfindungsgemässen Polymeren mit verbesserten rheologischen Eigenschaften erfolgt oft erst nach einer gewissen Adaptationszeit der Mischkultur.

Als Komponente für Mischkulturen mit Pseudomonas-Arten, insbesondere Pseudomonas maltophilia, kommen insbesondere die folgenden Bakterienarten in Betracht:

Agrobacterium tumefaciens, Agrobacterium radiobacter, Agrobacterium rhizogenes, Agrobacterium rubi, Klebsiella pneumoniae, Klebsiella planticola, Enterobacter sp., Xanthomonas sp. und Bacillus polymyxa. «Enterobacter sp.» und «Xanthomonas sp.» stehen hierbei für alle Arten dieser Gattungen, welche als polysaccharidbildende Bakterien bekannt sind.

Mit den folgenden Bakterienstämmen wurden in Mischungen mit Pseudomonas maltophilia DSM 2130 Biopolymere erhalten, die eine besonders deutliche Erhöhung der Viskosität im Vergleich zum Produkt der Reinkultur zeigen:

| Bakterium | Viskositäts-erhöhung |
|---|---|
| Enterobacter sakazaki | 30% |
| Bacillus polymyxa | 40% |
| Klebsiella pneumoniae | 45% |
| Agrobacterium tumefaciens | 100% |

Besonders bevorzugt für eine Mischkultur mit Pseudomonas maltophilia DSM 2130 ist das Agrobacterium tumefaciens, das bei der Deutschen Sammlung von Mikroorganismen unter der Nummer DSM 2128 hinterlegt ist. Dieser Stamm sowie seine Mutanten, die ebenfalls in Mischkulturen extrazelluläre Polymere mit vorteilhaften Eigenschaften bilden, sind weiterhin Gegenstand der Erfindung. Die entsprechenden Mischkulturen können über mindestens 3 Monate in kontinuierlicher Kultur stabil gehalten werden.

Die Fermentation erfolgt in an sich bekannter Weise. Als Kohlenstoffquelle enthält das Fermentationsmedium monomere, dimere oder oligomere Zucker, wie beispielsweise Glukose, Saccharose oder Lactose oder eine beliebige Mischung dieser Zucker, wobei die Zuckerkonzentration im Medium 10 bis 100 g/l, vorzugsweise 40 bis 60 g/l, beträgt. Anstelle der reinen Zucker werden technisch besonders vorteilhaft Kohlenhydrate enthaltende Rohmaterialien, z.B. Flüssigzucker, Melasse, Molke oder Molkepulver eingesetzt, wobei die Zuckerkonzentration den vorstehend genannten Werten entspricht.

Als Stickstoffquelle im Fermentationsmedium kommen organische Produkte wie Sojamehl, Cornsteep (Maisquellwasser), Hefeextrakt, Weizenkleie sowie Harnstoff oder anorganische Salze wie Nitrate oder beliebige Ammoniumsalze oder beliebige Mischungen von zwei oder mehr der genannten Substrate in Betracht, wobei die Stickstoffkonzentration im Medium so gewählt wird, dass sie 0,5 bis 250 mM, vorzugsweise 10 bis 50 mM, $NH_3$ entspricht.

Der pH-Wert wird bei der Fermentation im Bereich von 5 bis 9, vorzugsweise 6 bis 7,5, gehalten.

Die Temperatur wird während der Anwachsphase für etwa 8 bis 12 Stunden bei etwa 35 bis 45 °C gehalten und danach bis zur Beendigung der Polymerbildung zwischen etwa 15 und 45 °C, vorzugsweise 20 bis 40 °C, insbesondere 25 bis 35 °C. Das Zuckersubstrat und die Stickstoffquelle werden entweder in der vollen Menge gleich zu Beginn der Fermentation zugegeben oder nach und nach dem Fermenter zudosiert. In diesem Falle wird vorteilhaft die Zuckerkonzentration im gesamten Verlauf der Fermentation zwischen 0,001 und 1 Gew.-% konstant gehalten und die Stickstoffquelle so zudosiert, dass während der Fermentation im Medium gleichbleibend eine Konzentration zwischen 0,0001 und 1 mM $NH_3$ herrscht.

Das extrazelluläre Polymer kann aus dem Fermentationsmedium durch Ultrafiltration und/oder Fällung isoliert werden, beispielsweise mit polaren Lösemitteln wie Aceton oder niederen Alkanolen oder mit Salzen wie Magnesiumchlorid oder Calciumchlorid und Basen wie Natriumhydroxid oder Kaliumhydroxid. Vor der Ausfällung kann das Produkt mit zellysierenden Enzymen wie Lysozym oder mit proteolytischen Enzymen wie Trypsin oder mit beiden nacheinander zur Klärung und Eiweissspaltung behandelt werden, wodurch ein besonders reines Produkt erhalten wird. Vor der Fällung des Polymers kann die Fermentationslösung auch mit Reagenzien versetzt werden, die Eiweiss und Nucleinsäuren ausfällen, worauf die Lösung von den ausgefällten Stoffen abgetrennt wird, beispielsweise durch Filtrieren oder Zentrifugieren.

Die Fermentation und die Isolierung des Produkts können jeweils diskontinuierlich oder kontinuierlich durchgeführt werden.

Erfindungsgemässe Polymere sind wie folgt zusammengesetzt:

Glucose und Galactose im Molverhältnis von etwa 4 : 1 bis 8 : 1 als Hauptkomponenten und als Nebenkomponenten etwa 4 bis 9 Gew.-% Pyruvat, etwa 5 bis 15 Gew.-% Succinat, etwa 0,5 bis 7 Gew.-% Rhamnose und etwa 0,2 bis 5 Gew.-% Mannose.

In den folgenden Beispielen wird die Erfindung näher erläutert. Prozentangaben und Verhältnisse beziehen sich auf das Gewicht, sofern nichts anderes angegeben ist.

Beispiel 1
Herstellung der Mischkultur

Zunächst werden die Stämme DSM 2128 und 2130 auf folgenden Medien getrennt kultiviert: 30 g Glucose, 3 g Cornsteep (trocken), 0,5 g $MgSO_4 \cdot 7$ $H_2O$, 1 g $KH_2PO_4$ und 5 g $CaCO_3$ für Stamm DSM 2130 und zusätzlich 1 g $NaNO_3$ für Stamm 2128, jeweils Leitungswasser ad 1 l. Für Agarkulturen wird den Medien 1,8% Agar zugesetzt. Die Bebrütungszeit für Agarkulturen beträgt 2 Tage bei 30 °C. Schüttelkulturen erfolgen in 300 ml-Kolben mit je 100 ml Füllung bei 30 °C auf einem Rundschüttler (Schütteldurchmesser 5 cm, Drehzahl 180 UpM), Brutzeit 24 Std.

Vor der Beimpfung einer Fermenterkultur werden die Kulturen beider Stämme 1 : 1 gemischt und in das genannte Medium mit $NaNO_3$ überimpft. Die angesetzte Mischkultur wird noch 2 bis 3 mal nach jeweils 24 Std. Wachstumszeit in das gleiche Medium geimpft und dabei stabilisiert. Das Mischungsverhältnis der beiden Arten ändert sich danach bei weiterer Überimpfung nicht mehr und bleibt bei regelmässigem Überimpfen mindestens 2 Jahre lang stabil.

Fermentation

Von der vorstehend beschriebenen stabilisierten Mischkultur werden 300 bis 500 ml als Inokulum für einen Fermenter mit 10 l Inhalt verwendet, der ein Fermentationsmedium der folgenden Zusammensetzung (pro l) enthält:

| 45 g Glucose | Spurenelementelösung: |
| 1,5 g $NaNO_3$ | pro l |
| 0,5 g $(NH_4)_2$ $HPO_4$ | 3 g $CaCl_2$ |
| 2 g $KH_2$ $PO_4$ | 1 g Fe(III)citrat |
| 1 g $Na_2HPO_4 \cdot 7\ H_2O$ | 0,2 g $MnSO_4$ |
| 0,5 g $MgSO_4 \cdot 7\ H_2O$ | 0,1 g $ZnCl_2$ |
| 0,5 g Hefeextrakt | 0,025 g $CuSO_4$ |
| 5 ml Spuren-elemente-lösung | 0,022 g $CoCl_2$ |
| | 0,025 g $Na_2MoO_4 \cdot 2\ H_2O$ |
| | 0,010 g Ethylendiamin-tetraessig-säure |

Die Fermentation erfolgt bei 30 °C, wobei der Inhalt mit einem Spiralrührer (450 UpM) gerührt wird. Die Durchlüftung erfolgt zu Beginn mit 10 l/min, nach etwa 24 Stunden mit steigender Viskosität wird die Luftmenge bis zu 18 l/min erhöht. Der pH wird auf 6,8 mit Salzsäure und Natronlauge eingeregelt. Nach 48 bis 60 Stunden ist die Glucose restlos abgebaut, wobei pro Liter Fermentationslösung 25 g Polymer gebildet wird.

Die Kulturlösung wird durch Erhitzen auf 85 °C vor der Aufarbeitung pasteurisiert und die Bakterienzellen nach Verdünnung abzentrifugiert. Anschliessend wird das Polymer durch Zugabe von Aceton ausgefällt, wobei etwa das 1,5-fache des Volumens der wässrigen Lösung erforderlich war. Das ausgefällte Produkt wird getrocknet; es enthält noch etwa 5% Eiweiss.

Anstelle der Zentrifugation können die Bakterienzellen nach bekannten Verfahren in der Fermentationslösung chemisch oder enzymatisch aufgelöst werden. Anstelle von Aceton kann die Fällung auch mit Isopropanol oder einem anderen niederen wasserlöslichen Alkanol erfolgen.

Wenn ein reineres Produkt gewünscht wird, können zunächst die Proteine und Nukleinsäuren in bekannter Weise ausgefällt und abgetrennt werden und hierauf das Polysaccharid ausgefällt werden, welches dann erneut in Wasser gelöst und abschliessend gefällt wird.

Die folgende Tabelle zeigt die Viskosität des so erhaltenen Produktes in Vergleich zu Xanthan Gum beim Schergefälle D = 10/sec und D = 424/sec in wässriger und salzhaltiger Lösung (13% NaCl + 1% CaCl$_2$) bei einer Konzentration von 0,2% (Angaben in mPa.s, bei 22 °C):

| Schergefälle D (s$^{-1}$) | Produkt in Wasser | Xanthan in Wasser | Produkt in Salzlösung | Xanthan in Salzlösung |
|---|---|---|---|---|
| 10 | 265 | 118,7 | 256 | 118,6 |
| 424 | 21 | 11 | 16,5 | 13 |

Das Polysaccharid ist aus folgenden Monomeren zusammengesetzt:

Glucose und Galactose im Verhältnis 6 : 1 bis 7 : 1 als Hauptkomponenten, 5,1 bis 7,9% Pyruvat, 6,4 bis 8,7% Succinat, 1 bis 1,5% Rhamnose und ca. 0,4% Mannose als Nebenkomponenten.

Die Elementaranalyse ergab: 41% C, 5,6% H, 45% O, 1,7% N, 0,8% P.

Beispiel 2

Die Fermentation erfolgt wie im Beispiel 1 beschrieben. Nach dem Pasteurisieren wird jedoch das Produkt wie folgt ausgefällt:

Pro 100 g Polymer werden 64 g Magnesiumchlorid zugegeben und aufgelöst. Anschliessend wird die Lösung mit 51 g Natriumhydroxid alkalisch gestellt, wobei das Magnesiumsalz des Polymers ausfällt. Das gefällte Produkt wird abfiltriert und mit einer Mischung aus 2 Vol.-Teilen Isopropanol und 1 Vol.-Teil 4 M Salzsäure gewaschen, wobei 15 l pro kg Fällungsprodukt verwendet werden. Anschliessend wird mit der gleichen Menge Isopropanol nachgewaschen und dann das von Magnesiumionen befreite Produkt getrocknet.

Anstelle der 64 g Magnesiumchlorid können auch 75 g Calciumchlorid eingesetzt werden, wobei dann das Calciumsalz des Polymers ausgefällt wird.

Eine weitere Reinigung kann wie im Beispiel 1 angegeben erfolgen.

Beispiel 3

Für eine kontinuierliche Fermentation wird das gleiche Medium verwendet wie in Beispiel 1. Nach einer Wachstumszeit von 20 Std. beginnt die kontinuierliche Nachdosierung des gleichen Mediums. Die Verdünnungsrate beträgt 0,04 pro Stunde. Die gleiche Menge wird kontinuierlich entnommen und der Aufarbeitung zugeführt. Alle beschriebenen Schritte der Aufarbeitung in Beispiel 1 werden jetzt kontinuierlich durchgeführt, jedoch entfällt das Pasteurisieren der Lösung nach Entnahme aus dem Fermenter. Die abzentrifugierte Zellmasse wird zu 90% in den Fermenter zurückgeführt und zu 10% pasteurisiert und verworfen. Die aus dem Fermenter entnommene Suspension enthält 20–22 g Polymerprodukt pro l und einen Restzuckergehalt unter 0,5%. Die Bakterienstamm-Mischung bleibt ohne regelnde Eingriffe über mindestens 3 Monate stabil.

**Patentansprüche**

1. Extrazelluläre Polysaccharide, enthaltend Glucose und Galactose im Molverhältnis von 4 : 1 bis 8 : 1 als Hauptkomponenten und als Nebenkomponenten etwa 4–9 Gew.-% Pyruvat, etwa 5–15 Gew.-% Succinat, etwa 0,5–7 Gew.-% Rhamnose und etwa 0,2–5 Gew.-% Mannose, erhältlich durch Fermentation einer Mischkultur aus zwei oder mehreren Mikroorganismen aus der Gruppe Agrobacterium, Alcaligenes, Arthrobacter, Aureobasidium, Azotobacter, Bacillus, Corynebacterium, Erwinia, Hansenula, Klebsiella, Leuconostoc, Methylococcus, Methylocystis, Methylomonas, Nocardia, Pseudomonas, Sclerotium, Streptomyces und Xanthomonas, von denen mindestens einer auch in Reinkultur ein Polysaccharid produziert.

2. Verfahren zur Herstellung von extrazellulären Polysacchariden durch Fermentation von Mikroorganismen, dadurch gekennzeichnet, dass eine Mischkultur aus zwei oder mehreren Mikroorganismen aus der Gruppe Agrobacterium, Alcaligenes, Arthrobacter, Aureobasidium, Azotobacter, Bacillus, Corynebacterium, Erwinia, Hansenula, Klebsiella, Leuconostoc, Methylococcus, Methylocystis, Methylomonas, Nocardia, Pseudomonas, Sclerotium, Streptomyces und Xanthomonas, von denen mindestens einer auch in Reinkultur ein Polysaccharid produziert, kultiviert wird, bis sich ein Polysaccharid, enthaltend Glucose und Galactose im Molverhältnis von etwa 4 : 1 bis 8 : 1 als Hauptkomponenten und als Nebenkomponenten etwa 4–9 Gew.-% Pyruvat, etwa 5–15 Gew.-% Succinat, etwa 0,5–7 Gew.-% Rhamnose und etwa 0,2–5 Gew.-% Mannose, im Nährmedium anhäuft.

3. Verfahren nach Anspruch 2, dadurch gekennzeichnet, dass einer der Mikroorganismen der Stamm Pseudomonas maltophilia DSM 2130 ist.

4. Verfahren nach Anspruch 2 oder 3, dadurch gekennzeichnet, dass einer der Mikroorganismen der Stamm Agrobacterium tumefaciens DSM 2128 ist.

5. Verfahren nach einem oder mehreren der Ansprüche 2–4, dadurch gekennzeichnet, dass das gebildete Polymer aus dem Fermentationsmedium durch Ultrafiltration und/oder Fällung isoliert wird.

6. Verwendung des Polysaccharids nach Anspruch 1, als Viskositätsregulator.

7. Pseudomonas maltophilia DSM 2130, Agrobacterium tumefaciens DSM 2128 sowie deren Mutanten, die in Mischkultur extrazelluläre Polysaccharide gemäss Anspruch 1 produzieren.

## Claims

1. Extracellular polysaccharides containing glucose and galactose in the molar ratio of 4 : 1 to 8 : 1 as main components and about 4–9% by weight of pyruvate, about 5–15% by weight of succinate, about 0.5–7% by weight of rhamnose and about 0.2–5% by weight of mannose as subsidiary components, obtainable by fermentation of a mixed culture of two or more microorganisms from the group comprising Agrobacterium, Alcaligenes, Arthrobacter, Aureobasidium, Azotobacter, Bacillus, Corynebacterium, Erwinia, Hansenula, Klebsiella, Leuconostoc, Methylococcus, Methylocystis, Methylomonas, Nocardia, Pseudomonas, Sclerotium, Streptomyces and Xanthomonas, of which at least one also produces a polysaccharide in pure culture.

2. A process for the preparation of extracellular polysaccharides by fermentation of microorganisms, which comprises cultivation of a mixed culture of two or more microorganisms from the group comprising Agrobacterium, Alcaligenes, Arthrobacter, Aureobasidium, Azotobacter, Bacillus, Corynebacterium, Erwinia, Hansenula, Klebsiella, Leuconostoc, Methylococcus, Methylocystis, Methylomonas, Nocardia, Pseudomonas, Sclerotium, Streptomyces and Xanthomonas, of which at least one also produces a polysaccharide in pure culture, until a polysaccharide containing glucose and galactose in the molar ratio of 4 : 1 to 8 : 1 as main components and about 4–9% by weight of pyruvate, about 5–15% by weight of succinate, about 0.5–7% by weight of rhamnose and about 0.2–5% by weight of mannose as subsidiary components accumulates in the nutrient medium.

3. The process as claimed in claim 2, wherein one of the microorganisms is the strain Pseudomonas maltophilia DSM 2130.

4. The process as claimed in claim 2 or 3, wherein one of the microorganisms is the strain Agrobacterium tumefaciens DSM 2128.

5. The process as claimed in one or more of claims 2–4, wherein the polymer formed is isolated from the fermentation medium by ultrafiltration and/or precipitation.

6. Use of the polysaccharide as claimed in claim 1 as viscosity regulator.

7. Pseudomonas maltophilia DSM 2130, Agrobacterium tumefaciens DSM 2128, and the mutants thereof, which in mixed culture produce extracellular polysaccharides as claimed in claim 1.

## Revendications

1. Polysaccharides extracellulaires, contenant du glucose et du galactose dans un rapport molaire d'environ 4 : 1 à 8 : 1 comme constituants principaux, et environ de 4 à 9% en poids de pyruvate, environ de 5 à 15% en poids de succinate, environ de 0,5 à 7% en poids de rhamnose et environ de 0,2 à 5% en poids de mannose, comme constituants secondaires que l'on peut obtenir par fermentation au moyen d'une culture mixte de deux ou plusieurs microorganismes choisis parmi le groupe formé par: Agrobacterium, Alcaligenes, Arthrobacter, Aureobasidium, Azotobacter, Bacillus, Corynebacterium, Erwinia, Hansenula, Klebsiella, Leuconostoc, Methylococcus, Methylocystis, Methylomonas, Nocardia, Pseudomonas, Sclerotium, Streptomyces et Xanthomonas, dont au moins l'un d'eux produit également un polysaccharide dans une culture pure.

2. Procédé de préparation de polysaccharides extracellulaires par fermentation de microorganismes, caractérisé en ce qu'on cultive une culture mixte de deux ou plusieurs microorganismes choisis parmi le groupe formé par: Agrobacterium, Alcaligenes, Arthrobacter, Aureobasidium, Azotobacter, Bacillus, Corynebacterium, Erwinia, Hansenula, Klebsiella, Leuconostoc, Methylococcus, Methylocystis, Methylomonas, Nocardia, Pseudomonas, Sclerotium, Streptomyces et Xanthomonas, dont au moins l'un d'eux produit également un polysaccharide dans une culture pure jusqu'à ce que s'accumule dans le milieu nutritif un polysaccharide contenant du glucose et du galactose dans un rapport molaire d'environ 4 : 1 à 8 : 1 comme constituants principaux, et environ de 4 à 9% en poids de pyruvate, environ de 5 à 15% en poids de succinate, environ de 0,5 à 7% en poids de rhamnose et environ de 0,2 à 5% en poids de mannose, comme constituants secondaires.

3. Procédé selon la revendication 2, caractérisé en ce qu'un des microorganismes est la souche Pseudomonas maltophilia DSM 2130.

4. Procédé selon la revendication 2 ou 3, caractérisé en ce qu'un des microorganismes est la souche Agrobacterium tumefaciens DSM 2128.

5. Procédé selon l'une ou plusieurs des revendications 2 à 4, caractérisé en ce que le polymère formé est isolé du milieu de fermentation par ultrafiltration et/ou précipitation.

6. Utilisation des polysaccharides selon la revendication 1 comme régulateurs de viscosité.

7. Pseudomonas maltophilia DSM 2130, Agrobacterium tumefaciens DSM 2128, ainsi que leurs mutants, qui produisent des polysaccharides extracellulaires selon la revendication 1, dans une culture mixte.